# EUROPEAN PATENT SPECIFICATION

(11) **EP 4 308 047 B1**
(45) Date of publication and mention of the grant of the patent: **11.03.2026**
(21) Application number: 22729934.4
(22) Date of filing: 18.05.2022
(51) Int. Cl.: A61F 2/966

(54) **LUMEN APPOSING STENT TO DELIVER TARGETED THERAPY**
LUMENVERBINDENDER STENT ZUR VERABREICHUNG EINER GEZIELTEN THERAPIE
ENDOPROTHÈSE DE CONNECTION DE LUMIÈRE POUR ADMINISTRER UNE THÉRAPIE CIBLÉE

(30) Priority: 19.05.2021 US 202163190491 P
(43) Date of publication of application: 24.01.2024
(60) Divisional application: 26151197.6
(73) Proprietor: Boston Scientific Scimed, Inc., Maple Grove, MN 55133 (US)
(72) Inventor: WALSH, Kevin, Wellesly, Massachusetts 02482 (US); FLEURY, Sean P., Princeton, Massachusetts 01541 (US); CALLAGHAN, David, Ashland, Massachusetts 01721 (US); RICHARDS, Laura Emily, Worcester, Massachusetts 01609 (US); FAVREAU, John Thomas, Norwell, Massachusetts 01562 (US); SCUTTI, James J., Norwell, Massachusetts 02061 (US)
(74) Representative: Vossius & Partner Patentanwälte Rechtsanwälte mbB
(86) International application number: PCT/US2022/029800
(87) International publication number: WO 2022/245921

(56) References cited:
- EP-A1- 2 526 904
- WO-A1-99/40853
- CN-A- 106 420 129
- CN-A- 106 420 129
- US-A- 6 053 900
- US-A1- 2006 058 866
- US-A1- 2006 184 237
- US-A1- 2006 265 043
- US-A1- 2016 082 248
- US-A1- 2017 340 434

## Description

### FIELD

The present disclosure relates generally to the field of medical devices, systems, and methods for operatively delivering a functional and/or therapeutic agent to a body, such as to body tissue. More particularly, the present disclosure relates to medical devices, systems, and methods utilizing a device as a scaffold on which a functional and/or therapeutic agent may be deposited and delivered to a body, such as in situ.

### BACKGROUND

Various implantable devices are used for a number of treatment protocols and procedures. For instance, stents, such as self-expanding metal stents (SEMS), may be used within an anatomical area (*e.g.,* body lumen, passage, vessel, duct, etc.) to enable fluid communication from one area to another, such as through a body lumen, or from one anatomical structure (e.g., body lumen or cavity or organ) to another anatomical structure (e.g., body lumen or cavity or organ). Stents, such as lumen apposing stents, may be used to increase and improve access to different anatomical regions, such as within the gastrointestinal (GI) tract. Current uses of lumen apposing stents include drainage of pancreatic pseudocysts and antegrade access to the gallbladder. Stents may also be used to hold body tissues in apposition. One challenged faced when using stents is that various forces (e.g., as a result of the patient's natural motion or involuntary movements within the body, such as peristaltic movements along the GI tract) may increase a risk for migration of the stent. Migration of an implanted stent may result in fluid leakage, discomfort for the patient, and/or an additional procedure to replace the stent. Different devices, systems, and methods for reducing or preventing migration of a deployed stent thus would be welcome in the industry. Moreover, expansion of uses of stents, such as for closure of full thickness resections, by addressing various challenges presented by such procedures, would also be welcome in the industry. Finally, different secure and efficient mechanisms for applying agents or substances to the target / deployment site of the stent during or after deployment would be welcome in the industry as well.

US2006/184237A1 discloses a system for delivering a stent, a membrane impregnated with an agent is attached to the stent.

### SUMMARY

This summary of the disclosure is given to aid understanding, and one of skill in the art will understand that each of the various aspects and features of the disclosure may advantageously be used separately in some instances, or in combination with other aspects and features of the disclosure in other instances. No limitation as to the scope of the claimed subject matter is intended by either the inclusion or non-inclusion of elements, components, or the like in this summary.

The invention is directed to a system for maintaining a flow path in tissue is provided with a first sheath, a stent disposed within the first sheath, and a second sheath disposed about the first sheath. The stent is configured to transition between a first configuration and a second configuration. A functional and/or therapeutic agent is disposed between the first sheath and the second sheath. The functional and/or therapeutic agent is provided in a gel or liquid form and is distally extrudable from between the first sheath and the second sheath.

In some embodiments, the functional and/or therapeutic agent includes an agent selected from the group including of: an adhesive agent, a healing agent, a therapeutic agent.

In some embodiments, the stent is disposed about an inner member; and the first sheath is proximally longitudinally retractable with respect to the inner member, the inner member is distally longitudinally extendable with respect to the first sheath, or both, such that the stent is released from within the first sheath to transition to the second configuration.

In some embodiments, the first functional and/or therapeutic agent component is disposed between the first sheath and the second sheath, and the second functional and/or therapeutic agent component is disposed over the stent when disposed within the first sheath.

In accordance with another aspect of the present disclosure, a system is disclosed as having a stent including a lumen extending longitudinally therethrough, the stent configured to shift between a first configuration and a second configuration; and a functional and/or therapeutic agent. In some embodiments, in the second configuration, the stent defines a first retention member, a second retention member, and a saddle region extending therebetween, and when the stent is positioned within a body lumen in the second configuration, the first retention member, the second retention member, the saddle region, and the tissue wall of the lumen define a volume configured to retain the functional and/or therapeutic agent therein.

In some embodiments, the first retention member and the second retention member are configured to space the tissue away from the saddle region.

In some embodiments, the functional and/or therapeutic agent includes an agent selected from the group including of: an adhesive agent, a healing agent, a functional or therapeutic agent.

In some embodiments, the system further includes a fluid delivery device configured to inject the functional and/or therapeutic agent. In some embodiments, the fluid delivery device is configured to inject the functional and/or therapeutic agent through the tissue wall and into the volume defined by the stent and the tissue wall. In some embodiments, the fluid delivery device is configured to extend through one of the first retention member or the second retention member to inject the functional and/or therapeutic agent into the volume defined by the stent and the tissue wall.

In some embodiments, the stent further includes another functional and/or therapeutic agent disposed therealong.

In accordance with another aspect of the present disclosure, to which the claims are not specifically directed, a method for creating an anastomosis includes advancing a delivery system from a first lumen across first and second tissues and into a second lumen. In some embodiments, the delivery system includes a first sheath, an inner member, a stent disposed within the first sheath and about the inner member, a second sheath disposed about the first sheath, and a functional and/or therapeutic agent disposed between the first sheath and the second sheath. In some embodiments, the stent is configured to transition between a first configuration and a second configuration. In some embodiments, the method also includes proximally retracting the first and second sheath with respect to the inner member, distally extending the inner member with respect to the first sheath and second sheath, or both, such that a first end of the stent is extended distally outside the first sheath and forms the first retention member within the second body lumen, and the functional and/or therapeutic agent is disposed on a surface of the stent. In some embodiments, the method further includes proximally retracting the first sheath with respect to the inner member, distally extending the inner member with respect to the first sheath, or both, such that a second end of the stent is unconstrained from the first sheath and forms the second retention member within the first body lumen.

In some embodiments, the method further includes disposing the functional and/or therapeutic agent on the surface of the saddle region of the stent upon proximally retracting the first sheath with respect to the inner member, distally extending the inner member with respect to the first sheath, or both.

In some embodiments, the method further includes disposing the functional and/or therapeutic agent on the surface of the second retention member upon proximally retracting the first sheath with respect to the inner member, distally extending the inner member with respect to the first sheath, or both.

In some embodiments, the method further includes disposing an additional functional and/or therapeutic agent along a surface of the saddle region.

In some embodiments, disposing the additional functional and/or therapeutic agent includes injecting the additional functional and/or therapeutic agent via a needle.

In some embodiments, the inner member includes a cutting element disposed at an end thereof, and the method further includes forming an anastomosis across the first and second tissue with the cutting element before extending the first end of the stent distally outside the first sheath.

### BRIEF DESCRIPTION OF THE DRAWINGS

Non-limiting examples of the present disclosure are described with reference to the accompanying figures, which are schematic and not intended to be drawn to scale. In the figures, each identical or nearly identical component illustrated is typically represented by a single numeral. For purposes of clarity, not every component is labeled in every figure, nor is every component in each embodiment of the disclosure shown where illustration is not necessary to allow those of skill in the art to understand the disclosure. In the figures:
**FIGS. 1A-1B** illustrate end and side views of a system for deploying a stent with a functional and/or therapeutic agent according to one or more embodiments described herein.
**FIGS. 2A-2D** illustrate examples of aspects of a deployment of a stent with a functional and/or therapeutic agent according to one or more embodiments described herein.
**FIG. 3A** provides a cross-sectional view of a end-to-end anastomosis formed with a stent with a functional and/or therapeutic agent according to one or more embodiments described herein.
**FIG. 3B** provides a cross-sectional view of a stent with a functional and/or therapeutic agent bridging apposed tissue walls according to one or more embodiments described herein.
**FIGS. 4A** illustrates an example of a stent according to one or more embodiments described herein deployed in a body lumen.
**FIGS. 4B-4E** illustrate various examples of a stent as in **FIG. 4A** with a functional and/or therapeutic agent deployed thereon according to one or more embodiments described herein.
**FIG. 5A** illustrates a percutaneous injection of a functional and/or therapeutic agent along a surface of a deployed stent according to one or more embodiments described herein.
**FIG. 5B** illustrates an intraluminal or intraductal injection of a functional and/or therapeutic agent along a surface of a deployed stent according to one or more embodiments described herein.
**FIG. 6** illustrates a perspective view of an example of a deployed stent with a functional and/or therapeutic agent and retention prongs according to one or more embodiments described herein.
**FIG. 7** illustrates a perspective view of an example of a device useful for deploying one or both of a stent and/or a functional and/or therapeutic agent according to one or more embodiments described herein.
**FIGS. 8A-8B** illustrate top and perspective views of a delivery system end cap according to one or more embodiments described herein.

### DETAILED DESCRIPTION

The following detailed description should be read with reference to the drawings, which depict illustrative embodiments. The present disclosure is not limited to the particular embodiments described, as such embodiments may vary. The terminology used herein is for the purpose of describing particular embodiments only and is not intended to be limiting beyond the scope of the appended claims. Unless defined otherwise, all technical terms used herein have the same meaning as commonly understood by one of ordinary skill in the art to which the disclosure belongs. Finally, although embodiments of the present disclosure may be described with specific reference to medical devices and systems and procedures for treating the gastrointestinal system, it should be appreciated that such medical devices and methods may be used to treat tissues of the abdominal cavity, digestive system, urinary tract, reproductive tract, respiratory system, cardiovascular system, circulatory system, and the like. The structures and configurations, and methods of deploying, in order to stabilize, maintain, and/or otherwise facilitate fluid flow paths may find utility beyond treatments discussed herein.

As used herein, "proximal end" refers to the end of a device that lies closest to the user (medical professional or clinician or technician or operator or physician, etc., such terms being used interchangeably herein without intent to limit, and including automated controller systems or otherwise) along the device when introducing the device into a patient, and "distal end" refers to the end of a device or object that lies furthest from the user along the device during implantation, positioning, or delivery.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

As used herein, the conjunction "and" includes each of the structures, components, features, or the like, which are so conjoined, unless the context clearly indicates otherwise, and the conjunction "or" includes one or the others of the structures, components, features, or the like, which are so conjoined, singly and in any combination and number, unless the context clearly indicates otherwise.

All numeric values are herein assumed to be modified by the term "about," whether or not explicitly indicated. The term "about," in the context of numeric values, generally refers to a range of numbers that one of skill in the art would consider equivalent to the recited value (e.g., having the same function or result). In many instances, the term "about" may include numbers that are rounded to the nearest significant figure. Other uses of the term "about" (e.g., in a context other than numeric values) may be assumed to have their ordinary and customary definition(s), as understood from and consistent with the context of the specification, unless otherwise specified. The recitation of numerical ranges or values by endpoints includes all numbers within that range, including the endpoints (e.g. 1 to 5 includes 1, 1.5, 2, 2.75, 3, 3.80, 4, and 5), and fractions thereof.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used in connection with other embodiments whether or not explicitly described unless clearly stated to the contrary.

It is noted that the specification and figures may refer to examples of stents. It is presently contemplated that methods and features described herein are applied to stents as understood in a broad sense and apply to a variety of devices such as grafts, tissue scaffolds, other medical implants or self-expanding or expandable structures, etc. Reference to stents herein will be understood to include reference to such appropriate devices implicitly for the sake of brevity and without intent to limit.

Various procedures involve the creation and/or maintenance of a flow path through at least one body lumen and/or across tissues, for example, for the flow and/or drainage of fluid therethrough. A medical device such as a stent, graft, or the like may be used to hold open the flow path. In many procedures, a stent, graft, or other medical device for maintaining an open flow path may be implanted endoscopically. Various additional or alternative procedures involve use of a stent, graft, or other medical device to hold together portions of a body tissue (such as to heal a lesion) or to hold together apposed body tissues. Such procedures may be advantageously performed endoscopically, as endoscopic procedures can be less invasive than traditional open or lap surgery procedures. For example, an endoscope may be introduced into a body lumen without the need for extensive surgical incisions.

Various challenges have heretofore been encountered with such procedures. Natural motions of a patient may result in various forces being applied to an implanted medical device, which may increase a risk of the medical device to migrate. Additionally, or alternatively, the implantation of a medical device into a body may elicit an immune response from surrounding tissue. Any of these risks may result in undesirable irritation of tissue and/or discomfort for a patient. In the event of device migration, fluids may leak undesirably around the device, for example, into other areas of the body, which may contribute to further discomfort and/or risk for the patient. Subsequent procedures may be necessary to reposition or retrieve a dislodged or migrated medical device.

Accordingly, there is a need for improved systems to maintain a position of implanted medical devices and/or to prevent, reduce, or otherwise manage irritation of tissue surrounding the implants. More generally, there is a need for increased functionality of implanted devices, such as to permit application of a wider variety of functional and/or therapeutic agents to a deployment site of the device. It will be appreciated that terms such as application, deployment, delivery, provision, etc. (and conjugations and other grammatical forms thereof) of a functional and/or therapeutic agent may be used interchangeably herein without intent to limit unless otherwise indicated.

With the above considerations in mind, a variety of advantageous medical outcomes may be realized by the devices and/or methods of the present disclosure below.

For example, a stent may be deployed in or across body tissues or lumens with at least one functional and/or therapeutic agent selected for treating the tissue at the deployment site. In some embodiments, the agent provides a mechanical effect, such as aiding in or promoting adherence of the tissue to other tissue (e.g., to close a lesion or to form an anastomosis or otherwise to hold together apposed tissues) or to the stent (such as to hold the stent in place with respect to the deployment site to resist migration). In some embodiments, the agent provides a biological cellular therapeutic effect, such as hemostasis, facilitating healing, aiding in management of irritation and/or inflammation of the surrounding tissue, reducing discomfort, and/or reducing infection (e.g., such as including an antibacterial agent). Examples of agents with adhesive properties which may be used with devices, systems, and methods of the present disclosure include, without limitation, cyanoacrylate, LED cured adhesive, hydrogel, etc. Examples of agents with healing properties (e.g., tissue healing agents) include, without limitation, silk hydrogel, chitin hydrogel, growth factor gels, anti-inflammatory agents, antibacterial and other antimicrobial agents (including, without limitation, agents including biocidal and biostatic properties), antibiotic agents, hemostatic agents, sclerosing agents, discomfort-reducing agents (including, without limitation, antispasmodic agents, ketorolac, corticosteroids, narcotic analgesic agents, non-narcotic analgesic agents, local anesthetic agents, alpha-adrenergic blockers, and combinations thereof), anti-growth agents, growth-promoting agents, etc. Examples of agents with therapeutic and/or protective properties include, without limitation, chemotherapy agents, bioabsorbable gels as carriers for imaging and/or therapy agents, growth factors inhibitors, anti-cancer drugs (e.g., including agents such as alkylating agents, antimetabolites, antimitotics, hormones, immunosuppressives, etc.), etc. The functional and/or therapeutic agent may be provided in any desired form suitable for the intended purpose, such as a suspension, bound to a polymer, compounded into a coating, etc. Any appropriate delivery agent or carrier, such as a hydrogel, (e.g., Polyvinyl Alcohol (PVA), Sodium Alginate (SA), Carboxy Methylcellulose (CMC), Hyaluronic Acid (HA), Hydroxypropyl Methyl Cellulose, Hydroxyethyl Starch (HES), Xanthan Gum (XG), Gellan Gum, Gelatin, Agar, Polylactic Acid (PLA), Chitosan, Lecithin) may be used for such agents. The functional and/or therapeutic agent may be in any desired weight per volume of delivery agent or carrier as indicated to achieve the desired functional and/or therapeutic effect. For instance, the functional and/or therapeutic agent may be suspended or compounded in the delivery agent or carrier at a desired weight per volume of delivery agent or carrier. The injection force of the agent/gel, excluding the force of stent deployment, preferably is less than 60N. The viscosity at a desired shear rate preferably does not result in injection forces of over the clinically acceptable level for the procedure and should be less than 60N. Moreover, the delivery agent or carrier may be formulated to react to the environment in which it is placed, such as by absorbing water and swelling, or by releasing water and shrinking, or by otherwise affecting release of the functional and/or therapeutic agent therefrom. It will be appreciated that the above-listed agents may be any known or heretofore known agent in the art, the broad principles of the present disclosure not being limited by particular examples. Embodiments are not limited in this context. Methods and systems described herein may accommodate deployment of stents and one or more functional and/or therapeutic agents together or in separate steps. Percutaneous and intraductal deployment methods are presently contemplated.

The present disclosure provides devices, systems, and methods for providing a functional and/or therapeutic agent in association with a device or structure such as a stent. For the sake of convenience, reference is made to a stent without intent to limit, such reference being intended to encompass other devices and/or structures suitable for use in accordance with various principles of the present disclosure, such as any structure which may form or provide a scaffold for a functional and/or therapeutic agent to be applied thereto and to be maintained thereon for delivery and/or application to tissue at a treatment site.

Agents such as adhesive agents, healing agents, and/or therapeutic / protective agents may be delivered with or to the stent in accordance with various principles of the present disclosure. The functional and/or therapeutic agent may be applied to the surface of the stent as it is deployed. Additionally or alternatively, the stent may carry or be provided with (such terms, and other similar terms, being used interchangeably herein without intent to limit) a component of a functional and/or therapeutic agent, which may be inactive until activated, such as at the deployment site. Another component of a functional and/or therapeutic agent may be applied to the surface of such stent upon deployment of the stent so that the components of the agent may interact or be activated in situ at the deployment site (or shortly before deployment, such as during the deployment procedure, such as during delivery of the stent). Additionally or alternatively, the stent may be configured to accept or to receive a functional and/or therapeutic agent after the stent has been deployed, and preferably to retain such agent with respect to tissue along which the stent is deployed to achieve the desired functional and/or therapeutic effect with respect to the tissue. In some embodiments, a gel as a carrier for an active agent may be formed in situ. For instance, one component could be a polymer for a gel, and the other component could be a cross-linking agent that instantly forms a gel when the two components are combined, or the gel could be thermosensitive and form a gel when exposed to body temperature (e.g., tissue at body temperature).

One particular benefit of various principles of the present disclosure is the ability to deliver a functional and/or therapeutic agent that is not activated until deployment. For instance, various conditions at the deployment site (e.g., contact with body tissue at the deployment site, contact with material of the stent, exposure to body fluids at the deployment site, exposure to body temperature, etc.) may trigger activation of the functional and/or therapeutic agent, which may be delivered in an inactivated state. Another benefit is the ability to customize a given stent for the therapeutic needs of the patient by selecting one or more of a variety agents to be used in conjunction with a stent not otherwise initially bearing or carrying or otherwise being associated with a particular agent, thereby expanding potential uses of a given stent.

In accordance with various principles of the present disclosure, a stent is configured to provide a region for retaining a functional and/or therapeutic agent against or adjacent tissue to be treated. For instance, the stent may have a longitudinal extent when in a deployed configuration defining a first retention member along a first end, a second retention member along a second end, and an intermediate region between the first and second retention members. The first retention member has a first diameter, the second retention member has a second diameter, and the intermediate region has an intermediate diameter less than both the first diameter and the second diameter. As such, a functional and/or therapeutic agent may be retained or contained between the first and second retention members and along the intermediate region. For instance, a functional and/or therapeutic agent may be provided or deployed or deposited (such terms, and similar other terms, begin used interchangeably herein without intent to limit), and retained within a volume extending about the intermediate region, between the retention members, and between the stent and tissue along which the stent is positioned, to provide a desired effect with respect to the tissue.

The various principles of the present disclosure may be applied to any of a variety of configurations of stents. For instance, principles of the present disclosure may be applied to stents configured for draining an anatomical region, or for opening or creating or maintaining or treating a flow path, or for holding together apposed tissue (such as to close a wound or lesion or to form an anastomosis), or for other therapeutic purposes. In some embodiments, the stent may simply provide a structure or scaffold for the functional and/or therapeutic agent. Principles of the present disclosure may be applied to expand or improve or enhance usage of stents in conjunction with full thickness resections of tissue, such as by using the stent to effect closure of the resected tissue.

A delivery and deployment system for delivering and deploying a stent and accompanying functional and/or therapeutic agent may include a handle with a first control knob for a first sheath positioned over the stent, a second control knob for a second sheath positioned over the first sheath, and a third control knob for an inner member on which the stent is deployed, each control knob capable of independent movement of the other control knob. A first port may be provided to access a guide lumen or channel through the inner member, such as for delivery of guidewire (e.g., to assist in navigating to the desired deployment site) or needle or injectable material (e.g., imaging materials a fluoroscopy agent such as a contrast agent). A second port may be provided to deliver a functional and/or therapeutic agent to the stent, including, without limitation, to the deployment site before the stent is delivered, over the stent as it is being deployed, over an already-deployed stent, and/or adjacent an already deployed stent.

The detailed description should be read with reference to the drawings, which are not necessarily to scale, which depict illustrative embodiments and are not intended to limit the scope of the invention.

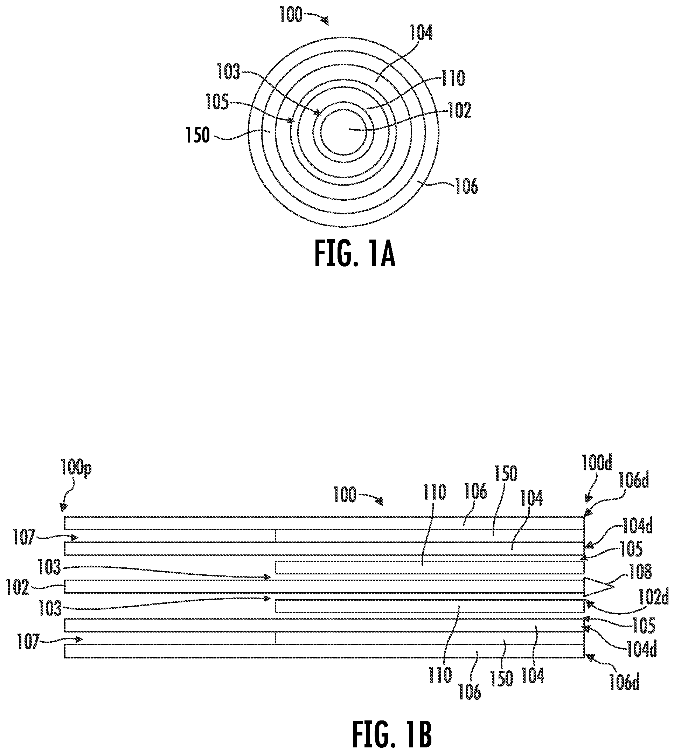

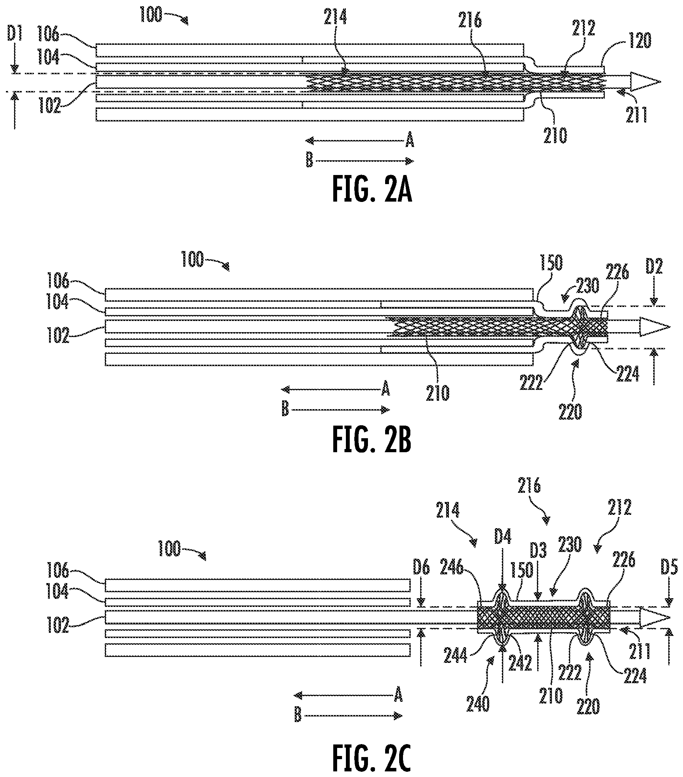

Turning now to the drawings, as illustrated in **FIGS. 1A-1B****,** various systems according to the present disclosure, including deployment system **100,** includes a stent **110** positioned between an inner member **102** and a first sheath **104** of the deployment system **100.** Stent **110** is configured to shift between a first configuration (e.g., collapsed configuration, constrained configuration, elongate configuration, delivery configuration, or the like) and a second configuration (e.g., expanded configuration, unconstrained configuration, deployed configuration, or the like). For the sake of simplicity and without intent to limit, a first configuration as described herein will be understood to additionally or alternatively refer to a constrained configuration, elongate configuration, delivery configuration, or the like, and a second configuration as described herein will be understood to additionally or alternatively refer to an unconstrained configuration, expanded configuration, deployed configuration, or the like. Stent **110** is illustrated in **FIGS. 1A-1B** in a first configuration but may have a second configuration with one or more similarities to the second configuration of stent **210** as illustrated in **FIGS. 2B-2D****.**

Stent **110** may be solid, woven, knit, or other recognizable form of stent, and may be made of metal, polymer, silicone, or other material. In various embodiments, stent **110** may be formed of a shape memory material. For example, stent **110** may be formed of one or more shape memory wires, such as Nitinol, Platinol, and the like alloys. Stent **110** may be preset to a second configuration and subsequently constrained within first sheath **104.** Various known or heretofore known covers (not illustrated for the sake of simplicity in the drawings), such as polymeric or elastomeric or silicone or lubricious coatings, may be applied to selected regions of the stent **110.** It will be appreciated that terms such as covers, coatings, sheaths, etc., may be used interchangeably herein without intent to limit terms. Provision of a cover may contribute to the mechanics of stent **110,** impart structural stability, occlude (partially or fully) flow of materials through the walls of the stent **110,** and/or inhibit tissue ingrowth. Selected regions of the stent **110** may be remain uncoated to permit tissue ingrowth to resist migration of the stent **110,** with optionally-coated regions resisting collapse and migration, thus facilitating sufficient immobility to allow for ingrowth into the uncoated regions.

Stent **110** may be disposed between first sheath **104** and inner member **102** such that stent **110** is held within the first configuration. A first intervening space **103** and/or a second intervening space **105** may exist between stent **110** and the respective inner member **102** or first sheath **104.** It will be appreciated that first intervening space **103** and/or second intervening space **105** are optional and may not be large enough to enable stent **110** to loosely rest within first sheath **104.** In various embodiments, inner member **102** and first sheath **104** may hold stent **110** securely therebetween.

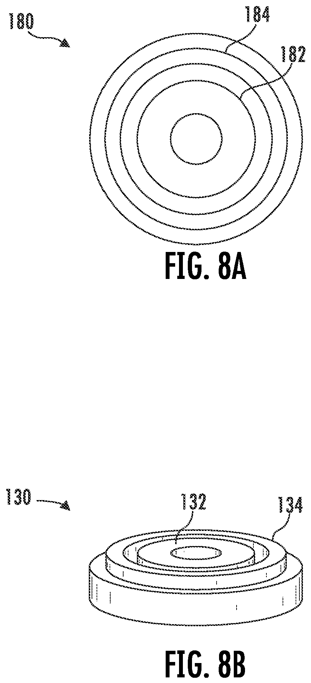

A second sheath **106** is be disposed about first sheath **104.** A functional and/or therapeutic agent **150** is be disposed in a space **107** between first sheath **104** and second sheath **106.** Functional and/or therapeutic agent **150** is in the form of a gel or liquid (i.e., a fluid). In any event, but particularly in examples in which functional and/or therapeutic agent **150** comprises a fluid, a cap **180** as illustrated in **FIG. 8** (and described in further detail below) may be provided along distal end **100d** of deployment system **100** such as by being fitted to distal end **104d** of at least first sheath **104** and/or distal end **106d** of second sheath **106** prior to a procedure and/or until a practitioner is ready to release functional and/or therapeutic agent **150.** Alternatively or additionally, a membrane may be provided along distal end **100d** of deployment system **100,** the membrane being thin enough or otherwise configured to be puncturable by a stylet passed through space **107,** breached by pressure of an agent, or dissolvable (e.g., at body temperature).

In various embodiments, functional and/or therapeutic agent **150** may comprise adhesive and/or healing and/or other treatment (e.g., tissue treatment) properties. For example, functional and/or therapeutic agent **150** may comprise one or more of the following: a glue or sealant or other agent with adhesive properties (e.g., cyanoacrylate, LED cured adhesive, hydrogel, etc); various healing hydrogels / gels (e.g., silk hydrogel, chitin hydrogel, growth factor gels) and/or other healing agents (anti-inflammatory agents); growth factors and/or inhibitors; therapeutic and/or protective agents (e.g., chemotherapy and/or radiation therapy agents); or the like, such as described above. Embodiments are not limited in this context.

In some embodiments, a cutting element **108** may be positioned at or on a distal end **102d** of inner member **102.** For example, cutting element **108** may comprise an electrocautery tip, a blade or sharpened needle tip, or the like, which may be configured to puncture or cut through tissue. The cutting element **108** may be used to create a pathway through tissue through which the stent **110** may be extended. In some embodiments, a lumen or channel is formed through inner member **102** through which a guidewire may be passed and used to guide the stent **110,** inner member **102,** first sheath **104,** and second sheath **106** to the deployment site. Additionally or alternatively, such lumen or channel may be used to deliver a needle, an injectable, etc., therethrough.

In various embodiments, one or both of first sheath **104** and second sheath **106** may be distally longitudinally extendable (towards distal end **100d** of deployment system **100)** and/or proximally longitudinally retractable (towards proximal end **100p** of deployment system **100)** with respect to inner member **102** and/or to each other. Inner member **102** may be distally longitudinally extendable and/or proximally longitudinally retractable with respect to first sheath **104** and/or second sheath **106.** In various embodiments, distal longitudinal extension of inner member **102** with respect to first sheath **104** and/or proximal longitudinal retraction of first sheath **104** with respect to inner member **102** may allow stent **110** to transition from its first configuration (when within first sheath **104)** to its second configuration upon exiting or being unsheathed from first sheath **104.**

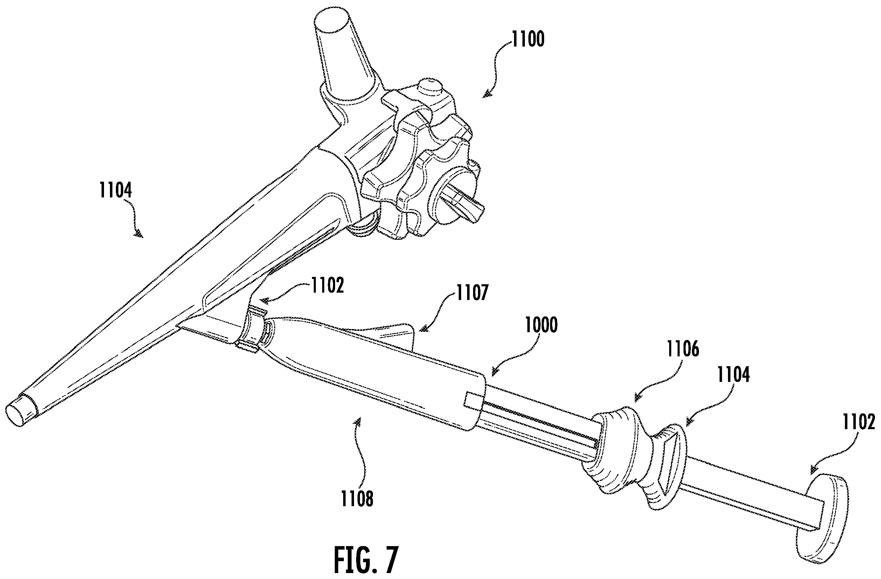

Additionally, or alternatively, as second sheath **106** is proximally retracted together with or with respect to first sheath **104,** functional and/or therapeutic agent **150** may be unsheathed from second sheath **106** and/or moved distally relative to distal end **106d** of second sheath **106** and/or moved distally relative to distal end **104d** of first sheath **104.** According to the invention, the functional and/or therapeutic agent **150** is distally extruded or extended from between first sheath **104** and second sheath **106.** For instance, functional and/or therapeutic agent **150** may be moved distally with respect to distal end **104d** of first sheath **104** and distal end **106d** of second sheath **106** such as a result of pressure applied thereto from a proximally positioned pushing member (not shown), or a proximal source of air pressure, or other force which may be controlled via a control handle **1000** coupled to proximal end **100p** of deployment system **100** (such as to a proximal end of one or more of inner member **102,** first sheath **104,** or second sheath **106).** For example, a control handle **1000** such as illustrated in **FIG. 7** (and described in further detail below) may comprise means to affect a pressurization of air proximal to functional and/or therapeutic agent **150** (e.g., into space **107),** thereby effecting a distal extrusion of the functional and/or therapeutic agent **150.**

Various aspects of an example of deployment of an example of an embodiment of a stent **210** are illustrated in **FIGS. 2A-2D****.** Although stent **210** is illustrated as being formed of a braided wire, various alternative known and heretofore known constructions and/or configurations of stents are within the scope of the present disclosure. Of note, stent **210** may shift or transition between first and second configurations as described with respect to stent **110** and may be similarly disposed between inner member **102** and first sheath **104** as described with respect to **FIGS. 1A-1B****,** with inner member **102** extending through a lumen **211** extending longitudinally through stent **210.** In some embodiments, a cover may be provided over stent **210,** such as described with respect to stent **110.**

The example of a stent **210** illustrated in **FIG. 2A** has a first end **212,** a second end **214,** and a middle segment **216** extending therebetween, which may have the same or substantially the same diameters **D1** in the first configuration. As may be seen in **FIGS. 2A-2B****,** as first sheath **104** and second sheath **106** are proximally retracted in the proximal direction of arrow **A** with respect to inner member **102** and stent **210,** and/or as inner member **102** (carrying stent **210)** is distally extended in the distal direction of arrow **B** with respect to first sheath **104** or both first sheath **104** and second sheath **106,** first end **212** of stent **210** may transition to a second configuration of stent **210** which may define a first retention member **220** along first end **212** of stent **210.** In some examples, diameter **D2** of first retention member **220** (of a portion of stent **210** in a second configuration) is greater than diameter **D1** of first end **212** of stent **210** in the first configuration. In various embodiments, first retention member **220** may, in some examples, be a double-walled retention member having a first retention member inner wall **222** facing middle segment **216** (e.g., facing proximally) and a first retention member outer wall **224** facing away from middle segment **216** (e.g., facing distally), and generally coupled to first retention member inner wall **222,** either or both of which may be configured to interface with tissue.

First sheath **104** and second sheath **106** may be further proximally retracted in the proximal direction of arrow **A** with respect to inner member **102,** and/or inner member **102** may be distally extended in the distal direction of arrow **B** with respect to first sheath **104** or both first sheath **104** and second sheath **106,** such that middle segment **216** forms a saddle region **230** extending proximally from first retention member **220** in the second configuration. Saddle region **230** may be generally cylindrical. Saddle region **230** may have the same or different diameter (e.g., diameter **D3)** as middle segment **216** in the first configuration (e.g., diameter **D1).** In several examples, saddle region **230** may have a larger diameter than middle segment **216** in the first configuration.

As may be seen in **FIG. 2C****,** first sheath **104** and second sheath **106** may be even further proximally retracted in the proximal direction of arrow **A** with respect to inner member **102,** or inner member **102** may be distally extended in the distal direction of arrow **B** with respect to first sheath **104,** or both first sheath **104** and second sheath **106,** such that second end **214** forms a second retention member **240** proximally adjacent to saddle region **230.** In some examples, diameter **D4** of second retention member **240** (of a portion of stent **210** in the second configuration) may be greater than diameter **D1** of second end **214** of stent **210** in the first configuration. Second retention member **240** may, in some examples, be a double-walled retention member having a second retention member inner wall **242** (e.g., facing proximally) and second retention member outer wall **244** facing away from middle segment **216** (e.g., facing distally), either or both of which may be configured to interface with tissue.

First retention member **220** and second retention member **240** may have identical or different shapes, sizes, and/or configurations. For example, diameter **D2** and diameter **D4** may be equal in magnitude or different. In various examples, one or both of first retention member **220** and/or second retention member **240** may include a flange, for example, a double-walled flange. In other examples, first retention member **220** and/or second retention member **240** may include a ridge, bump, flare, ramp, cylindrical portion with a greater diameter than the diameter of saddle region **230,** or other surface feature (not shown), which may extend fully or partially around a circumference of the stent **210.** In several embodiments according to the present disclosure, diameter **D2** and diameter **D4** may be greater than a diameter of a cutting element (not shown, but such as cutting element **108** illustrated in **FIG. 1B****)** such that first retention member **220** and second retention member **240** may be wider than an anastomosis formed by a cutting element.

In some examples, stent **210** may include a first lip **226,** which may extend from the first retention member **220** away from saddle region **230** (e.g., extend distally). Similarly, stent **210** may include a second lip **246,** which may extend from the second retention member **240** away from saddle region **230.** Diameter **D5** of first lip **226** and diameter **D6** of second lip **246** may be the same or different diameter, and may be the same or different from diameter **D3** of saddle region **230.** In some embodiments, diameter **D5** and/or diameter **D6** is larger than diameter **D3** so that stent **210** has wider openings through retention members **220, 240** at its respective ends **212, 214** than the diameter of the portion of lumen **211** extending through saddle region **230,** which may facilitate greater flow of fluid through lumen **211** extending through stent **210** when in the second configuration.

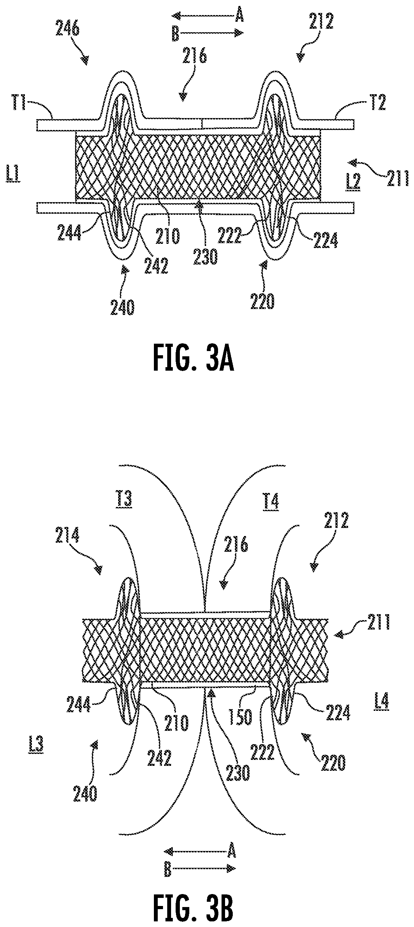

As may be seen with reference to the example illustrated in **FIGS. 2A-2C****,** functional and/or therapeutic agent **150** may be deployed and disposed along stent **210** as stent **110** deployed. For example, a positive pressure applied from proximal end **100p** of deployment system **100** into space **107** between first sheath **104** and second sheath **106,** such as actuated by handle **1000** (illustrated in **FIG. 7****),** may be exerted on the functional and/or therapeutic agent **150,** optionally in coordination with a proximal retraction of first sheath **104** and second sheath **106** with respect to inner member **102,** and/or in coordination with a distal extension of inner member **102** with respect to first sheath **104** and second sheath **106,** such that functional and/or therapeutic agent **150** is disposed along a portion of stent **210** (e.g., along one or more of first end **212,** middle segment **216,** or second end **214)** as stent **210** is distally released from within or unsheathed from first sheath **104.** In some examples and as illustrated in **FIGS. 2A-2C****,** a layer of functional and/or therapeutic agent **150** may thus be deposited along a portion of or along an entire outer surface of stent **210** (such as illustrated, for example, in **FIG. 3A** or **FIG. 3B****).**

In other examples, a positive pressure applied from proximal end **100p** of deployment system **100** into space **107** between first sheath **104** and second sheath **106** may be effected via handle **1000** (illustrated in **FIG. 7****)** so as to selectively and/or partially dispose functional and/or therapeutic agent **150** along stent **210.** For example, functional and/or therapeutic agent **150** may be disposed such that an entirety or part of one or more of lip **226,** first retention member outer wall **224,** first retention member **220,** first retention member inner wall **222,** saddle region **230,** second retention member inner wall **242,** second retention member **240,** second retention member outer wall **244,** or lip **246** is not covered with a layer of functional and/or therapeutic agent **150** once deployed. In other words, functional and/or therapeutic agent **150** may intermittently or discontinuously or partially cover stent **210.** For example, stent **210** as illustrated in **FIG. 3B** includes functional and/or therapeutic agent **150** disposed only along saddle region **230.** In other embodiments, not illustrated for the sake of simplicity (such concepts being readily understood by those of ordinary skill in the art), functional and/or therapeutic agent **150** may be disposed along each of first retention member **220** and second retention member **240** but not along saddle region **230,** along saddle region **230** and first retention member inner wall **222** and second retention member inner wall **242** only, along lip **226** and first retention member **220,** along lip **246** and second retention member **240,** or along any other combination(s) of lip **226,** first retention member outer wall **224,** first retention member **220,** first retention member inner wall **222,** saddle region **230,** second retention member inner wall **242,** second retention member **240,** second retention member outer wall **244,** or lip **246.**

It will be understood that, in the event that stent **210** comprises a cover (not shown for the sake of simplicity in the drawings), the cover may be helpful in preventing functional and/or therapeutic agent **150** from flowing through one or more holes of a porous surface (e.g., gaps in the braided weave) of stent **210** and into lumen **211.** Accordingly, a cover may be helpful in maintaining a position of functional and/or therapeutic agent **150** along the surface of stent **110.** Stent **210** may comprise a full cover or a partial cover. For example, a partial cover may include one or more segments of a cover along its length in areas in which a presence of functional and/or therapeutic agent **150** is desirable, with remaining portions of stent **210** not comprising a cover (thus allowing functional and/or therapeutic agent **150** to flow or drain through a porous surface of stent **210,** for example, or functional and/or therapeutic agent **150** not being provided along such portion of stent **110).** In some embodiments, one or more markers along stent **210** may indicate a start or an end of a partial cover (not shown) or other region along stent **210** so as to indicate where functional and/or therapeutic agent **150** should be disposed. Accordingly, excess use of functional and/or therapeutic agent **150** may be minimized, which may reduce an amount of functional and/or therapeutic agent **150** needed for an initial loading of functional and/or therapeutic agent **150** between first sheath **104** and second sheath **106,** and/or which may minimize an amount of functional and/or therapeutic agent **150** which flows into a patient's body. In some embodiments, the functional and/or therapeutic agent **150** may be laid down before and/or after positioning and deployment of the stent **210,** such as to act as a buffer (e.g., for regions of lesions that might be longer than stent **210).**

Functional and/or therapeutic agent **150** may be disposed along one or more portions of stent **210** such that a respective diameter along stent **210** is increased in magnitude above diameter **D1,** diameter **D2,** diameter **D3,** diameter **D4,** diameter **D5,** or diameter **D6,** although in various embodiments functional and/or therapeutic agent **150** may be disposed along stent **210** so as to not substantially increase a respective diameter of stent **210.** Embodiments are not limited in this context.

Functional and/or therapeutic agent **150** may in some embodiments comprise a curable agent such as an adhesive, which may cure automatically over time, such as with exposure to biological tissue or body temperature, or based on an external stimulus. For example, functional and/or therapeutic agent **150** may cure based on an exposure to UV light, for example, from a light extended through a working channel of control handle **1000** as illustrated in **FIG. 7****.**

In some embodiments, functional and/or therapeutic agent **150** may comprise multiple components, which, when in contact with each other, effect a curing and/or polymerization process. For example, functional and/or therapeutic agent **150** may comprise a fibrin glue, in which a first component may comprise thrombin and a second component may comprise fibrinogen. In some examples in which functional and/or therapeutic agent **150** comprises multiple components, particularly which may be reactive with each other, a first functional and/or therapeutic agent component **152** may be disposed in space **107** between first sheath **104** and second sheath **106** (such as in an inactive state), and a second functional and/or therapeutic agent component **154** may be disposed along a surface of stent **210** prior to deployment.

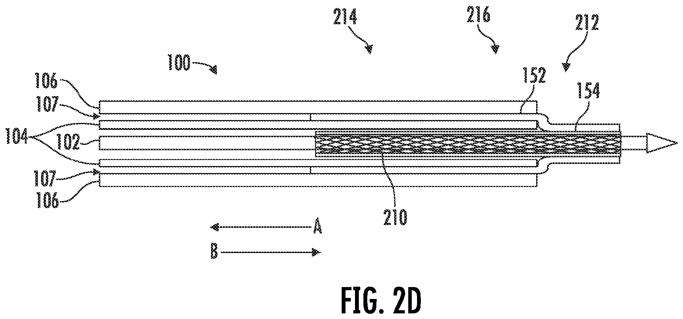

As illustrated in **FIG. 2D****,** rather than a complete, active form of functional and/or therapeutic agent **150** (such as described with respect to **FIGS. 2A-2C****),** an example of a first functional and/or therapeutic agent component **152** is illustrated disposed in space **107** between first sheath **104** and second sheath **106,** and may be disposed (e.g., deposited) along stent **210** similarly as functional and/or therapeutic agent **150** along stent **210** as described above with respect to **FIGS. 2A-2C****.** However, unlike the example illustrated in **FIGS. 2A-2C****,** stent **210** has second functional and/or therapeutic agent component **154** along all or a portion thereof (first end **212,** second end **214,** and/or middle segment **216)** before deployment. Accordingly, as first functional and/or therapeutic agent component **152** is deposited over stent **210** first functional and/or therapeutic agent component **152** may be deposited over second functional and/or therapeutic agent component **154** already in place over stent **110,** and first functional and/or therapeutic agent component **152** and second functional and/or therapeutic agent component **154** may react with each other, such as to polymerize in-situ. Embodiments are not limited in this context.

While not shown in **FIGS. 2A-2D** for the sake of simplicity, stents **210** may be deployed across one or more tissues, body lumens, or the like, such as to create a flow path therebetween and/or to join tissues. For example, as shown in **FIG. 3A****,** an example of an embodiment of a stent **210,** such as illustrated in the examples of **FIGS. 2A-2D****,** may be used to create end-to-end anastomoses. As shown in **FIG. 3B****,** an example of an embodiment of a stent **210,** such as illustrated in the examples of **FIGS. 2A-2D****,** may be used to create side-to-side anastomoses.

As illustrated in **FIG. 3A****,** stent **210** may be deployed from deployment system **100** (such as described with respect to **FIGS. 2A-2D****)** to extend across and through body lumen **L1** and body lumen **L2,** respectively defined by tissue **T1** and tissue **T2.** For example, tissue **T1** and tissue **T2** may be blood vessels, sections of intestine, or other tissue. In some embodiments, deployment of a first retention member **220** as described above may be within body lumen **L2.** For example, deployment system **100** may be introduced through body lumen **L1** and extended to or into body lumen **L2** (not shown). In this example, first sheath **104** and second sheath **106** may be proximally retracted with respect to inner member **102** while all three are disposed within body lumen **L2,** and/or inner member **102** may be distally extended with respect to first sheath **104** and second sheath **106** into body lumen **L2.** In any event, first end **212** may transition into first retention member **220** within body lumen **L2,** particularly such that first retention member **220** engages with an inner surface of tissue **T2,** thereby lodging, securing, anchoring, or otherwise retaining a position of a first end **212** of stent **210** within body lumen **L2.** Deployment system **100** may be proximally retracted (together with first end **212),** for example, in the direction of arrow **A,** such that tissue **T2** is moved with deployment system **100** and stent **210** and brought into apposition with tissue **T1.** Next, second retention member **240** may be deployed within body lumen **L1,** for example, via steps described above, such that second retention member **240** contacts tissue **T1** so as to be secured, lodged, or otherwise retained within body lumen **L1.** Functional and/or therapeutic agent **150** may serve, in many cases, to increase a retentive strength of the anastomosis facilitated by stent **210,** such as by adhering to one or both of tissue **T1** or tissue **T2** and/or causing tissue **T1** and tissue **T2** to adhere to each other (such as in examples in which tissue **T1** and tissue **T2** form an end-to-end anastomosis).

In the example illustrated in **FIG. 3B****,** stents **210** may be used to facilitate drainage from one organ or body lumen into another organ or lumen. For example, a cutting element **108** (such as illustrated in **FIG. 1B****)** may be distally advanced in the direction of arrow **B** from body lumen **L3,** through tissue **T3,** through tissue **T4,** and into body lumen **L4.** First retention member **220** may then be deployed within body lumen **L4** according to one or more aspects described above. In some examples, deployment system **100** (together with first retention member **220)** is distally advanced such that tissue **T4** is brought into apposition with tissue **T3.** Next, second retention member **240** may be deployed within body lumen **L3,** for example, via steps described above, such that second retention member **240** contacts tissue **T3** so as to be secured, lodged, or otherwise retained within body lumen **L3.** In some embodiments, functional and/or therapeutic agent **150** may adhere to one or both of tissue **T3** or tissue **T4,** such as to hold tissue **T3** and tissue **T4** in apposition with each other and stent **110.** For instance, saddle region **230** may extend across tissue **T3** and tissue **T4** and functional and/or therapeutic agent **150** along saddle region **230** may adhere to both tissue **T3** and tissue **T4.**

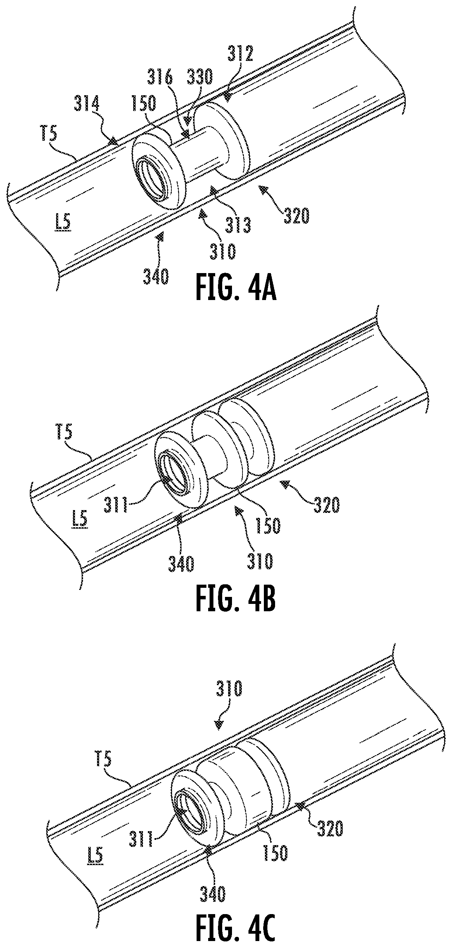

It will be appreciated that principles of the present disclosure with respect to delivery and deployment of a functional and/or therapeutic agent **150** need not be limited to anastomoses. For example, as illustrated in **FIGS. 4A-4E****,** a stent **310** may be positioned within a body lumen **L5,** such as an intact or single body lumen **L5,** to deliver or otherwise to provide a functional and/or therapeutic agent **150** with respect to tissue. For instance, an obstruction in a lumen may be opened by a stent **510,** and/or a functional and/or therapeutic agent **150** may be delivered to an affected area (e.g., diseased, damaged, etc.) along a lumen. Stent **310** may share one or more features or aspects of stents **110, 210** described above. For instance, a cover (such as a silicone cover or coating) may be provided over stent **310.** Stent **310** may have a first end **312** along which a first retention member **320** may be formed or provided, a second end **314** along which a second retention member **340** may be formed or provided, and a middle segment **316** along which a saddle region **330** extends. A functional and/or therapeutic agent **150** may be associated with stent **310.** In some embodiments, functional and/or therapeutic agent **150** is provided along saddle region **330** and longitudinally between first retention member **320** and second retention member **340,** and stent **310** is disposed with functional and/or therapeutic agent **150** positioned along tissue **T5** with an area to be treated. The functional and/or therapeutic agent **150** may be associated with stent **310** in any of a variety of manners as described above. For instance, functional and/or therapeutic agent **150** may be positioned on stent **310** as stent **310** is deployed, and/or may be provided in the form of one or more functional and/or therapeutic agent components, at least one of which is provided on stent **310** prior to deployment, such as prior to delivery, and at least another of which is provided on stent **310** during deployment to cause activation of functional and/or therapeutic agent **150** at the deployment site. Additionally or alternatively, functional and/or therapeutic agent **150** may be provided after stent **310** has been deployed. Stent **310** may be configured to hold additional, or alternative, functional and/or therapeutic agent **150** along its saddle region **330.** For example, first retention member **320** and second retention member **340** may hold tissue **T5** spaced away from saddle region **330** so as to create a working space into which functional and/or therapeutic agent **150** may be injected after deployment of stent **310,** for example, according to methods as discussed below.

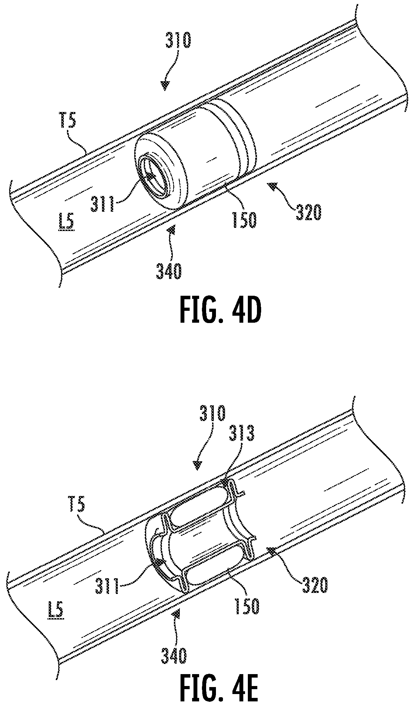

Functional and/or therapeutic agent **150** may be provided along saddle region **330** of stent **310** in incremental and/or predetermined volumes such that volume **313,** defined by saddle region **330,** first retention member **320,** second retention member **340,** and tissue **T5** (tissue forming the wall of lumen **L5** in which stent **310** is positioned), as illustrated in **FIG. 4A****,** may be filled with various percentages of functional and/or therapeutic agent **150.** For example, **FIGS. 4B** and **4C** illustrate respectively larger volumes of functional and/or therapeutic agent **150** injected along saddle region **330** between first retention member **320** and second retention member **340.** **FIG. 4D** illustrates an example in which substantially the entire volume **313** defined by saddle region **330,** first retention member **320,** second retention member **340,** and tissue **T5** has been filled with functional and/or therapeutic agent **150.** **FIG. 4E** illustrates a cross-sectional view of the example illustrated in **FIG. 4D****,** illustrating that lumen **311** of stent **310** may be maintained through stent **310,** for example, by the effectiveness of a cover over stent **310** retaining functional and/or therapeutic agent **150** within volume **313** and inhibiting flow of functional and/or therapeutic agent **150** through the stent **310** wall into lumen **311.** It will be appreciated that **FIGS. 4A-4E** may be viewed as sequentially illustrating different stages of delivery of a functional and/or therapeutic agent **150,** or each of **FIGS. 4A-4E** may be viewed as illustrating a stent **310** with a different volume of functional and/or therapeutic agent **150** already delivered thereto. In any or all of the examples of embodiments illustrated in **FIGS. 4A-4E****,** the functional and/or therapeutic agent **150** may be in any desired weight per volume of delivery agent or carrier as indicated to achieve the desired functional and/or therapeutic effect with the appropriate viscosity value. For instance, the functional and/or therapeutic agent **150** may be suspended or compounded in the delivery agent or carrier at a desired weight per volume of delivery agent or carrier.

In some embodiments, stent **310** may be disposed within a lumen **L5** having a tissue area to be treated. Saddle region **330** of stent **310** may be positioned along tissue **T5** with the tissue area to be treated positioned longitudinally between first retention member **320** and second retention member **340.** Functional and/or therapeutic agent **150** is provided in a gel or liquid form and may be contained within volume **313** and retained in place adjacent the tissue area to be treated. For instance, a cover over stent **310** (such as described above) may retain functional and/or therapeutic agent **150** within volume **313** without allowing functional and/or therapeutic agent **150** to seep out from volume **313.** In some embodiments, functional and/or therapeutic agent **150,** positioned within volume **313,** may comprise an agent as described above. For instance, functional and/or therapeutic agent **150** may include one or more healing agents, for example, growth factors, anti-inflammatory agents, or the like, which may aid in a healing of tissue **T5.** In some embodiments, functional and/or therapeutic agent **150** may comprise a first functional and/or therapeutic agent component **152** and a second functional and/or therapeutic agent component **154** delivered separately to the deployment site. For example, a second functional and/or therapeutic agent component **154** may be delivered with stent **310** and a first functional and/or therapeutic agent component **152** delivered separate from stent **310** and deployed with stent **310** such as into contact with the second functional and/or therapeutic agent component **154.** In some embodiments, additional amounts of functional and/or therapeutic agent **150,** or first functional and/or therapeutic agent component **152** and second functional and/or therapeutic agent component **154,** may be deployed into volume **313** after stent **310** has been deployed in lumen **L5** (as described in further detail below). Functional and/or therapeutic agent **150** may comprise at least one additionally adhesive component. For example, after deployment of stents **310,** a medical professional may determine that additional adhesion of stent **310** with respect to surrounding tissue may be desirable, in which case functional and/or therapeutic agent **150** may be provided after stent **310** has already been deployed, as described in further detail below. Embodiments are not limited in this context.

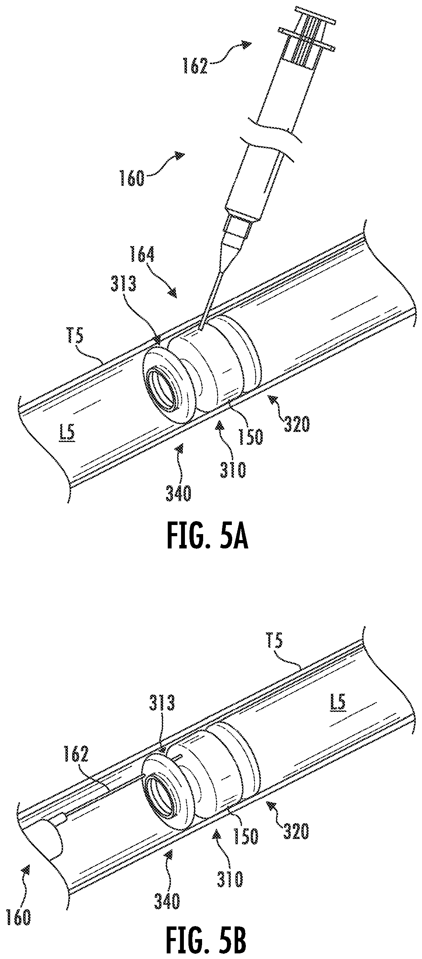

If functional and/or therapeutic agent **150,** or first functional and/or therapeutic agent component **152** and second functional and/or therapeutic agent component **154,** are not deployed with or when stent **310** is deployed, such as described above, a stent configured as stent **310** (e.g., with a retention member along each end and a saddle region therebetween) may permit later deployment of a functional and/or therapeutic agent or one or more components of a functional and/ or therapeutic agent with respect to the stent and the lumen in which the stent is positioned, such as stent **310** within lumen **L5** as illustrated in **FIG. 5A** and **FIG. 5B****.** In some embodiments, a functional and/or therapeutic agent or one or more components of functional and/or therapeutic agent may be injected into volume **313** defined by saddle region **330,** first retention member **320,** second retention member **340,** and tissue **T5.** As illustrated in **FIG. 5A****,** a fluid delivery device **160,** such as a syringe or hypodermic needle or the like, may be used to deliver functional and/or therapeutic agent **150** percutaneously through the wall of lumen **L5** and into volume **313** between the stent **310** and the lumen **L5.** Fluid delivery device **160** may be any desired shape or configuration and may have the necessary length to reach the treatment site at lumen **L5** percutaneously, as may readily be understood by those of ordinary skill in the art. For instance, functional and/or therapeutic agent may be dosed at a proximal dosage controller **162** and injected through tissue penetrating end **164** through the tissue **T5** and into volume **313.** Additionally or alternatively, in some embodiments, a functional and/or therapeutic agent or one or more components of a functional and/or therapeutic agent may be injected into volume **313** through a wall of stent **310,** such as illustrated in **FIG. 5B****.** For instance, a fluid delivery device **160** may be delivered transluminally through lumen **L5** to the deployment site of stent **310.** In some embodiments, the walls of stent **310** (such as the walls of one of the retention members **320, 340)** may be penetrable by tissue penetrating end **164** of fluid delivery device **160** so that a desired amount of functional and/or therapeutic agent may be dosed therethrough (e.g., from a proximal end of the fluid delivery device **160,** such as along a control handle **12** of a delivery and deployment device **10** as illustrated in **FIG. 7****).** As described above, a cover may be provided over stent **310** and may retain functional and/or therapeutic agent within volume **313.** Such cover may be penetrable by tissue penetrating end **164** of fluid delivery device **160** and self-sealing upon withdrawal of tissue penetrating end **164** to retain functional and/or therapeutic agent within volume **313.**

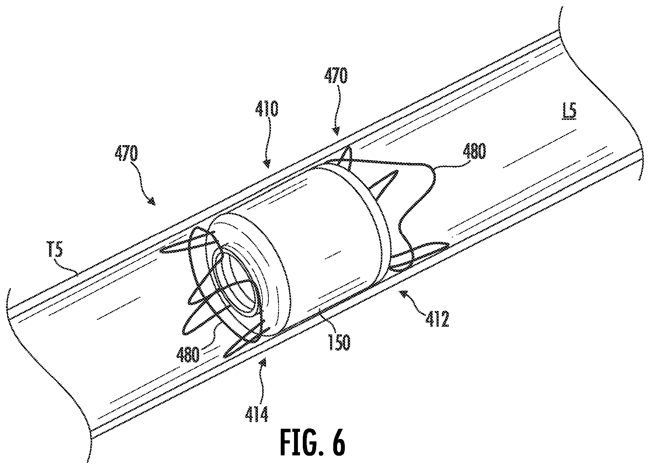

In some embodiments, as described above, a functional and/or therapeutic agent **150** with an adhesive agent may be provided within a volume **313** defined between stent **310** and lumen **L5** to inhibit migration of stent **310** with respect to tissue **T5.** In some embodiments, it may be desirable to provide mechanical retention or anti-migration features instead of or in addition to material or chemical-based retention agents (such as adhesive agents). For instance, functional and/or therapeutic agent **150** may not contain an adhesive agent, and/or additional retention features may be necessary if forces at the deployment site are thought at times to overcome adhesive properties of functional and/or therapeutic agent **150** and/or the retentive forces applied by the retention flanges of the stent. It will be appreciated that a stent as configured in any or all of **FIGS. 4A-4E****,** **5A, 5B** may be covered or coated to prevent seepage or leakage of a functional and/or therapeutic agent **150** deployed within the volume defined by the inner walls of the retention members and the outer surface of the saddle region thereof. Such cover generally resists tissue ingrowth, and thus may not inhibit migration of the stent. In accordance with various principles of the present disclosure in some embodiments, as illustrated in **FIG. 6****,** an embodiment of a stent **410** may be provided with additional mechanical anchoring elements. The anchoring elements may be in the form of one or more anchors **470** along stent **410,** such as along first end **412** and/or second end **414** of stent **410.** Anchors **470** may be in any desired form, such as loop-shaped projections or fins (as illustrated), or posts, quills, spikes, barbs, hooks, etc., such as known or heretofore known in the art to facilitate anchoring of a device, such as a stent, with respect to tissue to inhibit / prevent migration of the device relative to the tissue. Anchors **470** may be formed of resilient biocompatible materials such as metals or polymers, such as wires similar to those forming the stent **410.** Anchors **470** may be used to remove stent **410,** such as by pulling on one or more of anchors **470.** Additionally or alternatively, a removal / retrieval device **480,** such as a filament, a cord, a suture, a wire, a string, a band, etc., may be provided along or about a circumference of anchors **470** and/or an end **412, 414** of stent **410** to facilitate removal of stent **410,** if desired, in a manner known or heretofore known in the art. Retrieval device **480** may be configured and positioned with respect to stent **410** (e.g., interwoven with the wall of stent **410)** such that pulling of removal device **480** cinches or contracts stent **410** to facilitate removal of stent **410.** For instance, retrieval device **480** may be in the form of a suture retrieval loop that passes through both stent **410** and anchors **470** in a manner that pulls in anchors **470** and collapses stent **410** when retrieval device **480** is pulled, allowing stent **410** to be removed from the deployment site.

Deployment system **100** may be delivered to a treatment site via an endoscope **1100** such as illustrated in **FIG. 7****.** Endoscope **1100** may be any of a number of types of endoscopes or related medical devices, usually selected based on the particular anatomy desired to be reached, such as a colonoscope, duodenoscope, bronchoscope, gastroscope, or ureteroscope, or similar medical device. Deployment system **100** may inserted through a port **1102** in the handle **1104** of endoscope **1000** and a working channel through flexible elongate shaft **1108.** A deployable scaffold, such as known or heretofore known, may be provided at a distal end of elongate shaft **1108,** and may be positioned and subsequently expanded within a body lumen to improve access to and/or visualization of a target tissue / treatment site by the endoscope and/or a tool thereof. Endoscope **1100** may include any of a variety of visualization components, such as an optical cable connected to an external light source (not shown). An imaging element, such as a charge coupled device (CCD) camera can be disposed at the distal end to enable a user to visualize a working area. Various additional ports may be provided along endoscope handle **1104** for delivery of other tools, application of suction, delivery of fluid (e.g., air, water, saline, functional and/or therapeutic agents, etc.), etc., as known or heretofore known. Endoscope **1000** may comprise one or more additional controls, such as knobs, which may be operative to cause articulation of one or more components thereof. Articulation, for example, may include proximal retraction, distal extension, rotation about a longitudinal axis, movement along a lateral axis, movement along a transverse axis, or any combination thereof.

In general, the delivery and deployment device **10** may include a control handle **1000** to facilitate use of deployment system **100** for treating tissue according to embodiments described herein. Control handle **1000** may comprise one or more controls, such as actuators **1002, 1004, 1006,** which may be coupled, respectively, to inner member **102,** first sheath **104** and second sheath **106,** and operative to cause the desired distal advancement / extension or proximal retraction or other movement (e.g., rotation) or combination of movements of inner member **102,** first sheath **104** and second sheath **106.** An access port **1007** may be positioned along the control handle **1000** to facilitate introduction of pressure through the deployment system **100** to advance functional and/or therapeutic agent **150** (such as through space **107** between first sheath **104** and second sheath **106),** and/or to introduce additional functional and/or therapeutic agents or components thereof to a stent formed in accordance with various principles of the present disclosure. Additional ports for introducing pressure, fluids (e.g., for aspiration), or other agents (e.g., imaging materials such as contrast agents) may be provided as well, such as in fluid communication with a lumen through the inner member **102.**

As briefly described above, a cap **180** may be provided along distal end **100d** of deployment system **100** such as by being fitted to distal end **104d** of at least first sheath **104** and/or distal end **106d** of second sheath **106** prior to a procedure and/or until a practitioner is ready to release functional and/or therapeutic agent **150.** An example of an embodiment of a cap **180** is illustrated in **FIGS. 8A-8B** according to various embodiments of the present disclosure. Cap **180** may be made with a flexible and/or inflexible material, and may be disposable (e.g., single-use) or sterilizable and reusable.

In various embodiments, cap **180** may be configured to form an interference fit with one or more cavities at the distal end of deployment system **100.** For example, ridge **182** may be configured to form an interference fit between inner member **102** and first sheath **104** as described with respect to **FIGS. 1A-1B****,** and/or ridge **184** may be configured to form an interference fit between first sheath **104** and second sheath **106.** While not illustrated for the sake of simplicity in the drawings, alternatively configured attachment methods and mechanisms for caps are contemplated. For example, a cap may extend around a distal end of a deployment system **100** (e.g., around an outer diameter of second sheath **106)** and/or fit to one or more aspects of the distal end of the deployment system **100** via a screw fit, luer lock, or the like.

In various embodiments, cap **180** may be removed from a distal end of a deployment system **100** prior to introduction of the deployment system **100** into a body. In other embodiments, a deployment system **100** may be advanced into a body with cap **180** still coupled to its distal end, and cap **180** may be removed during a procedure, for example, by distal extension of inner member **102** with respect to first sheath **104** and/or second sheath **106.** Cap **180** may be subsequently removed from the body, for example, via a grasper (not shown). For example, if cap **180** is made of a sufficiently pliable material, cap **180** may be bent so as to have a smaller diameter than and be removable through a passage through stent **110** (through which inner member **102** extends) and through first sheath **104** and second sheath **106.** Alternatively, cap **180** may be bioabsorbable, or may be passed naturally through the body. Accordingly, cap **180** may be retained on an end of deployment system **100** until a time in a procedure at which disposition of functional and/or therapeutic agent **150** is desirable, although it will be understood that in some examples, functional and/or therapeutic agent **150** may be retained within deployment system **100** after removal of cap **180,** for example, by an effecting of a negative pressure proximally to functional and/or therapeutic agent **150** in working channel **16.** Embodiments are not limited in this context.

Embodiments described herein may comprise various dimensions for suitability for use in various applications. A device formed in accordance with various principles of the present disclosure may have a length, in a collapsed configuration, of at least about 14 mm, or as long as 40 mm, or as long as 60 mm, or as long as about 80 mm, or as long as about 100 mm, or as long as about 150 mm, or even as long as 200 mm, including lengths in increments of 1 mm therebetween. The diameter of a device formed in accordance with various principles of the present disclosure, when in a collapsed configuration, may be as small as about 3 mm, or even as small as about 2.5 mm, and as large as about 5 mm, or as large as about 6 mm, or as large as about 10 mm, including diameters in increments of 0.5 mm therebetween. In an expanded configuration, a device formed in accordance with various principles of the present disclosure may have a length of as short as about 14 mm, or as short as about 10 mm, and as long as about 35 mm, or as long as about 50 mm, or as long as about 60 mm, or as long as about 80 mm, or as long as about 100 mm, or as long as about 120 mm, or as long as about 140 mm, or as long as about 160 mm, or as long as about 180 mm, or even as long as about 200 mm, including lengths in increments of 1 mm therebetween. The diameter of the saddle of a device formed in accordance with various principles of the present disclosure, when in an expanded configuration, may be as large as about 20 mm, or as large as about 25 mm, or as large as about 30 mm, or as large as about 35 mm, or as large as about 40 mm, or even as large as about 60 mm, and may be as small as about 10 mm, or as small as about 6 mm, or even as small as about 3 mm, including diameters in increments of 0.5 mm therebetween. The length of a saddle of a device formed in accordance with various principles of the present disclosure, when in an expanded configuration, may be as long as about 10 mm, or as long as about 15 mm, or as long as about 20 mm, or as long as about 30 mm, or as long as about 35 mm, or even as long as about 200 mm, and may be as short as about 10 mm, or as short as about 5 mm, or even as short as about 3 mm, including lengths in increments of 1 mm therebetween. In the expanded configuration, the retention members of a device formed in accordance with various principles of the present disclosure may have a diameter of as large as about 21 mm, or as large as about 24 mm, or as large as about 29 mm, or as large as about 35 mm, or as large as about 40 mm, or even as large as about 60 mm, and as small as about 16 mm, or as small as about 14 mm, or even as small as about 5 mm; and a length of as long as about 6 mm, or as long as about 7 mm, or as long as about 10 mm, including diameters and lengths in increments of 0.5 mm therebetween, and as short as about 3 mm, or as short as about 2 mm, or even as short as about 0.5 mm. A device formed in accordance with various principles of the present disclosure may have a diameter, in an expanded configuration, which is at least about 1.5 times, and may be about 3 times, or as about 5 times, or as much as about 10 times, or even as much as about 15 times greater than a corresponding diameter of the device in a collapsed configuration. For instance, a 6 mm (length) stent may have a saddle diameter in a collapsed configuration of approximately 2.66 mm and in an expanded configuration of approximately 6 mm, with an expansion ratio of approximately 2.25; and a flange diameter in a collapsed configuration of approximately 2.66 mm and in an expanded configuration of approximately 14 mm, with an expansion ratio of approximately 5.26. A 20 mm (length) stent may have a saddle diameter in a collapsed configuration of approximately 3 mm and in an expanded configuration of approximately 20 mm, with an expansion ratio of approximately 6.66; and a flange diameter in a collapsed configuration of approximately 3 mm and in an expanded configuration of approximately 29 mm, with an expansion ratio of approximately 9.66. In devices with at least one lip along the retention members in accordance with various principles of the present disclosure, the lip length may be as short as about 1 mm and even as short as about 0.5 mm, and as long as about 1.5 mm, or as long as about 2.5 mm, or even as long as about 3 mm. In devices with at least one lip along the retention members in accordance with various principles of the present disclosure, the lip diameter may be at least about 1 mm wider or at least about 0.5 mm wider than the diameter of the saddle. In devices with at least one lip along the retention members in accordance with various principles of the present disclosure, the lip diameter may be as small as about 4 mm, or as small as about 6 mm, or as small as about 11 mm, and as large as about 22 mm, or as large as about 27 mm, including increments of 0.5 mm therebetween. For example, for a corresponding saddle diameter of about 10 mm, the lip diameter may be at least about 11 mm and at most about 14 mm; for a corresponding saddle diameter of about 15 mm, the lip diameter may be or at least about 16 mm and at most about 19 mm; and for a corresponding saddle diameter of about 20 mm, the lip diameter may be at least about 21 mm and at most about 24 mm, with all such dimensions including increments of 0.5 mm therebetween. In various embodiments of a stent with a lumen therethrough, the diameter of the lumen may be at least about 3 mm and at most about 60 mm, including diameters in increments of 0.5 mm therebetween. A device formed in accordance with various principles of the present disclosure may have a substantially constant diameter or a varying diameter along a longitudinal axis thereof. For example, a proximal end of a device formed in accordance with various principles of the present disclosure may have a proximal end with a smaller diameter than the diameter of the distal end, or larger diameters at the ends and a smaller diameter along the middle section. Various configurations and arrangements of sections which increase or decrease in diameter, or which have substantially constant diameters are within the scope of the present disclosure. Embodiments are not limited in this context

All of the devices and/or methods disclosed and claimed herein can be made and executed without undue experimentation in light of the present disclosure. While the system as defined in the claims has been described in terms of preferred embodiments, it may be apparent to those of skill in the art that variations can be applied without departing from the scope of the claims.

The foregoing discussion has broad application and has been presented for purposes of illustration and description and is not intended to limit the scope of the claims. It will be understood that various additions, modifications, and substitutions may be made to embodiments disclosed herein without departing from the scope of the claims. In particular, it will be clear to those skilled in the art that principles of the present disclosure may be embodied in other forms, structures, arrangements, proportions, and with other elements, materials, and components, without departing from the scope of the claims. For example, various features of the disclosure are grouped together in one or more aspects, embodiments, or configurations for the purpose of streamlining the disclosure. However, it should be understood that various features of the certain aspects, embodiments, or configurations of the disclosure may be combined in alternate aspects, embodiments, or configurations. While the disclosure is presented in terms of embodiments, it should be appreciated that the various separate features of the present subject matter need not all be present in order to achieve at least some of the desired characteristics and / or benefits of the present subject matter or such individual features. One skilled in the art will appreciate that the disclosure may be used with many modifications or modifications of structure, arrangement, proportions, materials, components, and otherwise, used in the practice of the disclosure, which are particularly adapted to specific environments and operative requirements without departing from the principles or scope of the present disclosure. For example, elements shown as integrally formed may be constructed of multiple parts or elements shown as multiple parts may be integrally formed, the operation of elements may be reversed or otherwise varied, the size or dimensions of the elements may be varied. Similarly, while operations or actions or procedures are described in a particular order, this should not be understood as requiring such particular order, or that all operations or actions or procedures are to be performed, to achieve desirable results. Additionally, other implementations are within the scope of the following claims. In some cases, the actions recited in the claims can be performed in a different order and still achieve desirable results. The presently disclosed embodiments are therefore to be considered in all respects as illustrative and not restrictive, the scope of the claimed subject matter being indicated by the appended claims, and not limited to the foregoing description or particular embodiments or arrangements described or illustrated herein. In view of the foregoing, individual features of any embodiment may be used and can be claimed separately or in combination with features of that embodiment or any other embodiment, the scope of the subject matter being indicated by the appended claims, and not limited to the foregoing description.

In the foregoing description and the following claims, the following will be appreciated. The phrases "at least one", "one or more", and "and/or", as used herein, are open-ended expressions that are both conjunctive and disjunctive in operation. The terms "a", "an", "the", "first", "second", etc., do not preclude a plurality. For example, the term "a" or "an" entity, as used herein, refers to one or more of that entity. As such, the terms "a" (or "an"), "one or more" and "at least one" can be used interchangeably herein. All directional references (e.g., proximal, distal, upper, lower, upward, downward, left, right, lateral, longitudinal, front, back, top, bottom, above, below, vertical, horizontal, radial, axial, clockwise, counterclockwise, and/or the like) are only used for identification purposes to aid the reader's understanding of the present disclosure, and/or serve to distinguish regions of the associated elements from one another, and do not limit the associated element, particularly as to the position, orientation, or use of this disclosure. Connection references (e.g., attached, coupled, connected, and joined) are to be construed broadly and may include intermediate members between a collection of elements and relative movement between elements unless otherwise indicated. As such, connection references do not necessarily infer that two elements are directly connected and in fixed relation to each other. Identification references (e.g., primary, secondary, first, second, third, fourth, etc.) are not intended to connote importance or priority, but are used to distinguish one feature from another.

In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous. In addition, singular references do not exclude a plurality. Reference signs in the claims are provided merely as a clarifying example and shall not be construed as limiting the scope of the claims in any way.

## Claims

1. A system (100) for maintaining a flow path in tissue, comprising:
a first sheath (104);
a stent (110) disposed within the first sheath (104), the stent configured to transition between a first configuration and a second configuration;
a second sheath (106) disposed about the first sheath (104); and
a functional and/or therapeutic agent (150), the functional and/or therapeutic agent disposed between the first sheath (104) and the second sheath (106),
the system being **characterized in that**
the functional and/or therapeutic agent (150) is provided in a gel or liquid form and is distally extrudable from between the first sheath (104) and the second sheath (106).

2. The system of claim 1, wherein the functional and/or therapeutic agent comprises an agent selected from the group consisting of: an adhesive agent, a healing agent.

3. The system (100) of any of claims 1-2, further comprising an inner member (102), wherein:
the stent (110) is disposed about an inner member (102); and
the first sheath (104) is proximally longitudinally retractable with respect to the inner member (102), the inner member is distally longitudinally extendable with respect to the first sheath (104), or both, such that the stent (110) is released from within the first sheath to transition to the second configuration.

4. The system (100) of claim 3, further comprising a cutting element (108) disposed at an end of the inner member (102).

5. The system of any of claims 3-4, wherein:
the second sheath (106) is proximally longitudinally retractable with respect to the inner member (102), or the inner member (102) is distally longitudinally extendable with respect to the second sheath (106), or both; and
the system is configured to dispose the functional and/or therapeutic agent (150) on the surface of the stent (110) when the second sheath (106) is proximally retracted with respect to the inner member (102), or when the inner member is distally extended with respect to the second sheath (106), or both.

6. The system (100) of claim 5, wherein the system is configured to dispose the functional and/or therapeutic agent (150) upon application of pressure from a proximal end of the system to the functional and/or therapeutic agent (150).

7. The system (100) of any of claims 5-6, wherein the functional and/or therapeutic agent comprises a first functional and/or therapeutic agent component (152) and a second functional and/or therapeutic agent component (154), the first component and the second component configured to react when in contact with each other.

8. The system (100) of claim 7, wherein the first functional and/or therapeutic agent component (152) is disposed between the first sheath (104) and the second sheath (106), and the second functional and/or therapeutic agent component (154) is disposed over the stent when disposed within the first sheath (104).

9. The system (100) of any of claims 1-8, wherein:
in the second configuration, the stent (110) defines a first retention member (220), a second retention member (240), and a saddle region (230) extending therebetween; and
when the stent (110) is positioned within a body lumen in the second configuration, the first retention member (220), the second retention member (240), the saddle region (230), and the tissue wall of the lumen define a volume (313) configured to retain the functional and/or therapeutic agent therein (150).

10. The system (100) of claim 9, wherein the first retention member (220) and the second retention member (220) are configured to space the tissue away from the saddle region (230).

## Patentansprüche

1. System (100) zur Aufrechterhaltung eines Strömungsweges in Gewebe, aufweisend:
eine erste Hülse (104);
einen Stent (110), der innerhalb der ersten Hülse (104) angeordnet ist, wobei der Stent zum Wechseln zwischen einer ersten Konfiguration und einer zweiten Konfiguration konfiguriert ist;
eine zweite Hülse (106), die um die erste Hülse (104) angeordnet ist; und
ein funktionelles und/oder therapeutisches Mittel (150), wobei das funktionelle und/oder therapeutische Mittel zwischen der ersten Hülse (104) und der zweiten Hülse (106) angeordnet ist,
wobei das System **dadurch gekennzeichnet ist, dass**
das funktionelle und/oder therapeutische Mittel (150) in Gel- oder Flüssigkeitsform vorliegt und von zwischen der ersten Hülse (104) und der zweiten Hülse (106) distal extrudierbar ist.

2. System nach Anspruch 1, wobei das funktionelle und/oder therapeutische Mittel ein Mittel umfasst, das ausgewählt ist aus der Gruppe bestehend aus: einem Haftmittel, einem Heilmittel.

3. System (100) nach einem der Ansprüche 1 - 2, ferner aufweisend ein Innenelement (102), wobei:
der Stent (110) um ein Innenelement (102) angeordnet ist; und
die erste Hülse (104) in Bezug auf das Innenelement (102) proximal in Längsrichtung zurückziehbar ist, das Innenelement in Bezug auf die erste Hülse (104) distal in Längsrichtung ausfahrbar ist oder beides, sodass der Stent (110) aus dem Inneren der ersten Hülse gelöst wird, um in die zweite Konfiguration zu wechseln.

4. System (100) nach Anspruch 3, ferner aufweisend ein Schneidelement (108), das an einem Ende des Innenelements (102) angeordnet ist.

5. System nach einem der Ansprüche 3 - 4, wobei:
die zweite Hülse (106) in Bezug auf das Innenelement (102) proximal in Längsrichtung zurückziehbar ist, oder das Innenelement (102) in Bezug auf die zweite Hülse (106) distal in Längsrichtung ausfahrbar ist oder beides; und
das System zum Verteilen des funktionellen und/oder therapeutischen Mittels (150) auf der Oberfläche des Stents (110) konfiguriert ist, wenn die zweite Hülse (106) in Bezug auf das Innenelement (102) proximal zurückgezogen ist, oder wenn das Innenelement in Bezug auf die zweite Hülse (106) distal ausgefahren ist oder beides.

6. System (100) nach Anspruch 5, wobei das System zum Verteilen des funktionellen und/oder therapeutischen Mittels (150) bei Anwendung von Druck von einem proximalen Ende des Systems auf das funktionelle und/oder therapeutische Mittel (150) konfiguriert ist.

7. System (100) nach einem der Ansprüche 5 - 6, wobei das funktionelle und/oder therapeutische Mittel eine erste funktionelle und/oder therapeutische Mittelkomponente (152) und eine zweite funktionelle und/oder therapeutische Mittelkomponente (154) aufweist, wobei die erste Komponente und die zweite Komponente zum Reagieren konfiguriert sind, wenn sie miteinander in Kontakt sind.

8. System (100) nach Anspruch 7, wobei die erste funktionelle und/oder therapeutische Mittelkomponente (152) zwischen der ersten Hülse (104) und der zweiten Hülse (106) angeordnet ist, und die zweite funktionelle und/oder therapeutische Mittelkomponente (154) über dem Stent angeordnet ist, wenn sie innerhalb der ersten Hülse (104) angeordnet ist.

9. System (100) nach einem der Ansprüche 1 - 8, wobei:
der Stent (110) in der zweiten Konfiguration ein erstes Rückhalteelement (220), ein zweites Rückhalteelement (240) und einen sich dazwischen erstreckenden Sattelbereich (230) definiert; und
das erste Rückhalteelement (220), das zweite Rückhalteelement (240), der Sattelbereich (230) und die Gewebewand des Lumens ein Volumen (313) definieren, das zum Zurückhalten des funktionellen und/oder therapeutischen Mittels (150) konfiguriert ist, wenn der Stent (110) innerhalb eines Körperlumens in der zweiten Konfiguration positioniert ist.

10. System (100) nach Anspruch 9, wobei das erste Rückhalteelement (220) und das zweite Rückhalteelement (220) zum Beabstanden des Gewebes von dem Sattelbereich (230) konfiguriert sind.

## Revendications

1. Système (100) pour maintenir une trajectoire d'écoulement dans le tissu, comprenant :
une première gaine (104) ;
un stent (110) disposé à l'intérieur de la première gaine (104), le stent étant configuré pour effectuer une transition entre une première configuration et une deuxième configuration ;
une deuxième gaine (106) disposée autour de la première gaine (104) ; et
un agent fonctionnel et/ou thérapeutique (150), l'agent fonctionnel et/ou thérapeutique étant disposé entre la première gaine (104) et la deuxième gaine (106),
le système étant **caractérisé en ce que** :
l'agent fonctionnel et/ou thérapeutique (150) est prévu sous une forme de gel ou de liquide et peut être extrudé, de manière distale, depuis l'endroit situé entre la première gaine (104) et la deuxième gaine (106).

2. Système selon la revendication 1, dans lequel l'agent fonctionnel et/ou thérapeutique comprend un agent sélectionné dans le groupe comprenant : un agent adhésif, un agent de cicatrisation.

3. Système (100) selon l'une quelconque des revendications 1 à 2, comprenant en outre un élément interne (102), dans lequel :
le stent (110) est disposé autour d'un élément interne (102) ; et
la première gaine (104) peut être rétractée de manière longitudinalement proximale par rapport à l'élément interne (102), l'élément interne est extensible de manière longitudinalement distale par rapport à la première gaine (104) ou les deux, de sorte que le stent (110) est libéré de l'intérieur de la première gaine afin d'effectuer une transition vers la deuxième configuration.

4. Système (100) selon la revendication 3, comprenant en outre un élément de coupe (108) disposé au niveau d'une extrémité de l'élément interne (102).

5. Système selon l'une quelconque des revendications 3 à 4, dans lequel :
la deuxième gaine (106) peut se rétracter de manière longitudinalement proximale par rapport à l'élément interne (102), ou bien l'élément interne (102) est extensible de manière longitudinalement distale par rapport à la deuxième gaine (106) ou les deux ; et
le système est configuré pour disposer l'agent fonctionnel et/ou thérapeutique (150) sur la surface du stent (110) lorsque la deuxième gaine (106) est rétractée de manière proximale par rapport à l'élément interne (102) ou bien lorsque l'élément interne est étendu, de manière distale, par rapport à la deuxième gaine (106) ou les deux.

6. Système (100) selon la revendication 5, dans lequel le système est configuré pour disposer l'agent fonctionnel et/ou thérapeutique (150), suite à l'application de pression à partir d'une extrémité proximale du système sur l'agent fonctionnel et/ou thérapeutique (150).

7. Système (100) selon l'une quelconque des revendications 5 à 6, dans lequel l'agent fonctionnel et/ou thérapeutique comprend un premier composant d'agent fonctionnel et/ou thérapeutique (152) et un deuxième composant d'agent fonctionnel et/ou thérapeutique (154), le premier composant et le deuxième composant étant configurés pour réagir lorsqu'ils sont en contact l'un avec l'autre.

8. Système (100) selon la revendication 7, dans lequel le premier composant d'agent fonctionnel et/ou thérapeutique (152) est disposé entre la première gaine (104) et la deuxième gaine (106), et le deuxième composant d'agent fonctionnel et/ou thérapeutique (154) est disposé sur le stent lorsqu'il est disposé à l'intérieur de la première gaine (104).

9. Système (100) selon l'une quelconque des revendications 1 à 8, dans lequel :
dans la deuxième configuration, le stent (110) définit un premier élément de retenue (220), un deuxième élément de retenue (240) et une région de selle (230) s'étendant entre eux ; et
lorsque le stent (110) est positionné à l'intérieur d'une lumière corporelle dans la deuxième configuration, le premier élément de retenue (220), le deuxième élément de retenue (240), la région de selle (230) et la paroi de tissu de la lumière définissent un volume (313) configuré pour retenir l'agent fonctionnel et/ou thérapeutique (150) dans ce dernier.

10. Système (100) selon la revendication 9, dans lequel le premier élément de retenue (220) et le deuxième élément de retenue (220) sont configurés pour éloigner le tissu de la région de selle (230).
